(19) Europäisches Patentamt
European Patent Office
Office européen des brevets

(11) Veröffentlichungsnummer: **0 375 613**
**A2**

(12) **EUROPÄISCHE PATENTANMELDUNG**

(21) Anmeldenummer: 89810945.9

(51) Int. Cl.5: **C07D 239/06, C07D 417/04, C07D 401/06, A01N 43/54**

(22) Anmeldetag: 12.12.89

Claims for the following Contracting State: ES.

(30) Priorität: **21.12.88 CH 4723/88**
**26.04.89 CH 1594/89**
**30.06.89 CH 2434/89**

(43) Veröffentlichungstag der Anmeldung:
**27.06.90 Patentblatt 90/26**

(84) Benannte Vertragsstaaten:
**AT BE CH DE ES FR GB IT LI NL**

(71) Anmelder: **CIBA-GEIGY AG**
**Klybeckstrasse 141**
**CH-4002 Basel(CH)**

(72) Erfinder: **Gsell, Laurenz, Dr.**
**Maiengasse 56**
**CH-4056 Basel(CH)**

(54) **Nitroenamine.**

(57) Die Erfindung betrifft neue Nitroenamine der Formel I

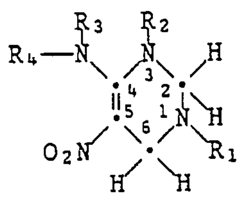

(I),

worin $R_1$ für Dialkylamino, Alkoxy, Alkenyloxy, Alkoxyalkyl, Alkylthioalkyl, Alkoxycarbonylamino, Aralkoxy, 2-Thiazolyl oder für die gegebenenfalls substituierte Reste aus der Reihe Alkyl, Alkenyl, Alkinyl, Cycloalkyl, Aralkyl und Hetarylalkyl steht,
$R_2$ für Nitro, Cyano, Hydroxy oder für gegebenenfalls substituierte Reste aus der Reihe Alkyl, Cycloalkyl, Alkenyl, Alkinyl, Aralkyl, Alkoxycarbonyl, Amino, Dialkylamino, Alkylcarbonyl, Alkoxycarbonyl, Alkylcarbonylamino, Alkoxycarbonylamino, Alkylcarbonyloxy und Pyridinylmethyl steht,
$R_3$ für Alkyl, Cycloalkyl oder für den gegebenenfalls substituierten Pyridinylmethylrest steht,
$R_4$ für Alkyl oder Cycloalkyl steht,
einschliesslich der Salze der Verbindungen der Formel I, Verfahren zur Herstellung dieser Verbindungen, sowie diese Wirkstoffe enthaltende Mittel und deren Verwendung in der Schädlingsbekämpfung, insbesondere zur Bekämpfung von Insekten.

EP 0 375 613 A2

## Nitroenamine

Die vorliegende Erfindung betrifft neue Nitroenamine, Verfahren zu ihrer Herstellung, Schädlingsbekämpfungsmittel, die diese Verbindungen enthalten, sowie ihre Verwendung in der Schädlingsbekämpfung.

Die erfindungsgemässen Nitroenamine entsprechen der Formel I

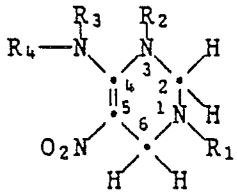

(I),

worin $R_1$ für Dialkylamino, Alkoxy, Alkenyloxy, Alkoxyalkyl, Alkylthioalkyl, Alkoxycarbonylamino, Aralkoxy, 2-Thiazolyl oder für die gegebenenfalls substituierte Reste aus der Reihe Alkyl, Alkenyl, Alkinyl, Cycloalkyl und Aralkyl steht,
$R_2$ für Nitro, Cyano, Hydroxy oder für gegebenenfalls substituierte Reste aus der Reihe Alkyl, Cycloalkyl, Alkenyl, Alkinyl, Aralkyl, Alkoxycarbonyl, Amino, Dialkylamino, Alkylcarbonyl, Alkoxycarbonyl, Alkylcarbonylamino, Alkoxycarbonylamino, Alkylcarbonyloxy und Pyridinylmethyl steht,
$R_3$ für Alkyl, Cycloalkyl oder für den gegebenenfalls substituierten Pyridinylmethylrest steht,
$R_4$ für Alkyl oder Cycloalkyl steht,
und umfassen auch die Salze der Verbindungen der Formel I.

Von den erfindungsgemässen Verbindungen werden jene bevorzugt, bei welchen $R_1$ Di-$C_1$-$C_5$-alkylamino, $C_1$-$C_5$-Alkoxy, $C_3$-$C_5$-Alkenyloxy, Methoxy-$C_1$-$C_4$-alkyl, Methylthio-$C_1$-$C_4$-alkyl, Ethoxycarbonylamino, $C_7$-$C_9$-Aralkoxy, 2-Thiazolyl oder eine der mit Halogen substituierten oder unsubstituierten Reste $C_1$-$C_5$-Alkyl, $C_3$-$C_5$-Alkenyl, $C_3$-$C_5$-Alkinyl, $C_3$-$C_7$-Cycloalkyl oder $C_7$-$C_9$-Aralkyl und ferner unsubstituiertes oder an der $\alpha$- oder $\beta$-Stelle durch Cyano, Carboxy, Aminocarbonyl oder $C_1$-$C_4$-Alkoxycarbonyl substituiertes $C_1$-$C_4$-Alkyl,
$R_2$ $C_1$-$C_5$-Alkyl, $C_3$-$C_7$-Cycloalkyl, oder, falls $R_3$ für Alkyl oder Cycloalkyl steht, den Rest Z

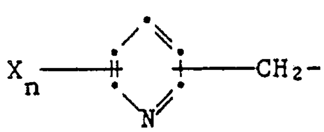

bedeutet, worin
X unabhängig voneinander für Chlor, Fluor, Nitro, Cyano, Hydroxy oder jeweils mit Halogen substituiertes oder unsubstituiertes $C_1$-$C_5$-Alkyl, $C_1$-$C_5$-Alkoxy, $C_1$-$C_5$-Alkylthio, $C_1$-$C_5$-Alkylsulfinyl oder $C_1$-$C_5$-Alkylsulfonyl und ferner $C_3$-$C_5$-Haloalkenyl, $C_3$-$C_5$-Haloalkinyl, $C_1$-$C_5$-Alkoxycarbonyl, Di-$C_1$-$C_4$-alkylamino, $C_1$-$C_5$-Alkylcarbonyl, $C_1$-$C_5$-Alkylcarbonylamino oder $C_1$-$C_5$-Alkylcarbonyloxy und n für die Zahlen 0 bis 4 steht,
$R_3$ $C_1$-$C_4$-Alkyl, $C_3$-$C_7$-Cycloalkyl oder den Rest Z, wie unter $R_2$ definiert und
$R_4$ $C_1$-$C_5$-Alkyl oder $C_3$-$C_7$-Cycloalkyl bedeuten.

Ebenfalls bevorzugt werden jene Verbindungen der Formel I, in welchen $R_1$ Dialkylamino, Alkoxy, Alkenyloxy, Aralkoxy oder einen gegebenenfalls substituierten Rest aus der Reihe Alkyl, Alkenyl, Alkinyl, Cycloalkyl, Aralkyl und Hetarylalkyl und
$R_2$ und $R_3$ voneinander unabhängig Alkyl, Cycloalkyl oder den gegebenenfalls substituierten Pyridinylmethylrest bedeuten.

Hiervon sind jene Verbindungen der Formel I hervorzuheben, in welchen $R_1$ Di-$C_1$-$C_5$-alkylamino, $C_1$-$C_5$-Alkoxy, $C_3$-$C_5$-Alkenyloxy, $C_7$-$C_9$-Aralkoxy oder eine der mit Halogen substituierten oder unsubstituierten Reste $C_1$-$C_5$-Alkyl, $C_3$-$C_5$-Alkenyl, $C_3$-$C_5$-Alkinyl, $C_3$-$C_7$-Cycloalkyl oder $C_7$-$C_9$-Aralkyl und ferner unsubstituiertes oder an der $\alpha$- oder $\beta$-Stelle durch Cyano, Carboxy oder $C_1$-$C_4$-Alkoxycarbonyl substituiertes $C_1$-$C_4$-Alkyl, $R_2$ $C_1$-$C_5$-Alkyl, $C_3$-$C_7$-Cycloalkyl, oder, falls $R_3$ für Alkyl oder Cycloalkyl steht, den Rest Z

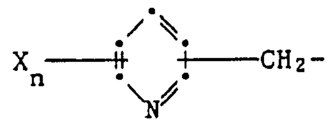

bedeutet, worin

X unabhängig voneinander für Chlor, Fluor, Nitro, Cyano, Hydroxy oder jeweils mit Halogen substituiertes oder unsubstituiertes $C_1$-$C_5$-Alkyl, $C_1$-$C_5$-Alkoxy, $C_1$-$C_5$-Alkylthio, $C_1$-$C_5$-Alkylsulfinyl oder $C_1$-$C_5$-Alkylsulfonyl und ferner $C_3$-$C_5$-Haloalkenyl, $C_3$-$C_5$-Haloalkinyl, Hydroxy, $C_1$-$C_5$-Alkoxycarbonyl, Di-$C_1$-$C_4$-alkylamino, $C_1$-$C_5$-Alkylcarbonyl, $C_1$-$C_5$-Alkylcarbonylamino oder $C_1$-$C_5$-Alkylcarbonyloxy steht,

n für die Zahlen 0 bis 4 steht,

$R_3$ $C_1$-$C_4$-Alkyl, $C_3$-$C_7$-Cyclalkyl oder den Rest Z, wie unter $R_2$ definiert und

$R_4$ $C_1$-$C_5$-Alkyl oder $C_3$-$C_7$-Cycloalkyl bedeuten.

Als Aralkyl kommen beispielsweise Benzyl, 1- oder 2-Phenylethyl in Betracht, als Aralkoxy können Reste wie Benzyloxy, 1- oder 2-Phenylethoxy genannt werden.

Die als Substituenten in Betracht kommenden Alkyle und alkylhaltigen Reste, wie Dialkylamino, Alkoxy, Alkoxycarbonyl, Alkylthio, Alkylsulfinyl, Alkylsulfonyl, Halogenalkyl, Cyanoalkyl, Alkylcarbonyl, Alkylcarbonylamino oder Alkylcarbonyloxy können geradkettig oder verzweigt sein. Als Beispiele solcher Alkyle seien Methyl, Aethyl, Propyl, Cyclopropyl, Isopropyl, Butyl, i-Butyl, sek.Butyl, tert.Butyl oder Pentyl, und ihre Isomeren genannt.

Als Cycloalkyle können beispielsweise Cyclopropyl, Cyclopentyl oder Cyclohexyl in Betracht kommen.

Bei den als Substituenten in Betracht kommenden Halogenen handelt es sich sowohl um Fluor und Chlor als auch um Brom und Jod, wobei Fluor und Chlor bevorzugt sind. Von den Alkylen sind jene mit 1 bis 5 Kohlenstoffatomen bevorzugt.

Die als Substituenten in Betracht kommenden durch Halogen substituierten $C_1$-$C_5$-Alkyle können geradkettig oder verzweigt sein und nur teilweise oder auch perhalogeniert sein, wobei für die Halogene und die Alkyle die oben gegebenen Definitionen gelten. Geeignete Beispiele solcher Substituenten sind u.a. das ein- bis dreifach durch Fluor, Chlor und/oder Brom substituierte Methyl wie z.B. $CHF_2$ oder $CF_3$; das ein- bis fünffach durch Fluor, Chlor und/oder Brom substituierte Aethyl wie z.B. $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie z.B. $CH_2CHBrCH_2Br$, $CF_2CHFCF_3$, $CH_2CF_2CF_3$ oder $CH(CF_3)_2$; das ein- oder mehrfach durch Fluor, Chlor und/oder Brom substituierte Butyl oder eines seiner Isomeren wie z.B. $CF(CF_3)CHFCF_3$ oder $CH_2(CF_2)_2CF_3$.

Als Pyridinylmethylrest kommt in den Verbindungen der Formel I jedes der drei Isomere α, β und γ der Formeln:

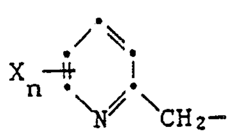

α

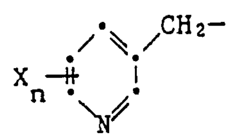

β

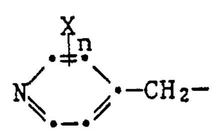

γ

in Betracht.

Die Verbindungen der Formel I können auch in Form von Säureadditionssalzen vorliegen. Zur Bildung solcher Salze eignen sich sowohl organische als auch anorganische Säuren. Beispiele solcher Säuren sind u.a. Chlorwasserstoffsäure, Bromwasserstoffsäure, Salpetersäure, verschiedene Phosphorsäuren, Schwefelsäure, Essigsäure, Propionsäure, Buttersäure, Valeriansäure, Oxalsäure, Malonsäure, Maleinsäure, Fumarsäure, Milchsäure, Weinsäure, Citronensäure oder Salicylsäure.

Im Rahmen der vorliegenden Erfindung sind in der Schädlingsbekämpfung jene Verbindungen der Formel I bevorzugt, bei welchen $R_1$ Cyclopropyl, ferner unsubstituiertes oder an der α- oder β-Stelle durch Cyano, Carboxy oder $C_1$-$C_4$-Alkoxycarbonyl substituiertes $C_1$-$C_4$-Alkyl,

$R_2$ Pyridin-3-ylmethyl, 6-Chlor-pyridin-3-ylmethyl, 2,6-Dichlor-pyridin-3-ylmethyl, 5,6-Dichlor-pyridin-3-ylmethyl, 6-Trifluormethyl-pyridin-3-yl-methyl oder Pyridin-4-ylmethyl und

$R_3$ und $R_4$ voneinander unabhängig $C_1$-$C_5$-Alkyl oder $C_3$-$C_7$-Cycloalkyl bedeuten.

Von dieser Gruppe sind einerseits jene Verbindungen insbesondere bevorzugt, bei welchen $R_2$ 6-Chlorpyridin-3-ylmethyl bedeutet, andererseits jene, bei welchen

$R_3$ Methyl und

$R_4$ Methyl, Aethyl, Isobutyl oder Cyclopropyl bedeuten.

Von den Verbindungen der Formel I sind in der Schädlingsbekämpfung auch jene bevorzugt, bei welchen $R_1$ Cyclopropyl, unsubstituiertes oder an der α- oder β-Stelle durch Cyano, Carboxy oder $C_1$-$C_4$-Alkoxycarbonyl substituiertes $C_1$-$C_4$-Alkyl,
$R_2$ $C_1$-$C_5$-Alkyl oder $C_3$-$C_7$-Cycloalkyl,
$R_3$ Pyridin-3-ylmethyl, 6-Chlor-pyridin-3-ylmethyl, 2,6-Dichlor-pyridin-3-ylmethyl, 6-Trifluormethyl-pyridin-3-ylmethyl oder Pyridin-4-ylmethyl und
$R_4$ $C_1$-$C_5$-Alkyl oder $C_3$-$C_7$-Cycloalkyl bedeuten.

Von dieser Gruppe sind jene hervorzuheben, bei welchen $R_1$ Cyclopropyl, unsubstituiertes oder an der α- oder β-Stelle durch Cyano, Carboxy, Methoxycarbonyl oder Aethoxycarbonyl substituiertes $C_1$-$C_4$-Alkyl,
$R_2$ Methyl, Aethyl, Isobutyl oder Cyclopropyl,
$R_3$ Pyridin-3-ylmethyl, Pyridin-4-ylmethyl oder 6-Chlor-pyridin-3-ylmethyl und
$R_4$ Methyl, Aethyl oder Cyclopropyl bedeuten.

Hervorzuheben sind dabei wegen ihrer Aktivität gegen Schädlinge die folgenden Einzelverbindungen:
das 1-Aethyl-3-(pyridin-3-ylmethyl)-4-dimethylamino-5-nitro-1,2,3,6-tetrahydropyrimidin,
das 1-Isobutyl-3-(pyridin-3-ylmethyl)-4-dimethylamino-5-nitro-1,2,3,6-tetrahydropyrimidin,
das 1-Aethyl-3-(pyridin-3-ylmethyl)-4-[(methyl-)(isobutyl)]-amino-5-nitro-1,2,3,6-tetrahydropyrimidin,
das 1-Isopropyl-3-(pyridin-3-ylmethyl)-4-[(methyl-)(isobutyl)]-amino-5-nitro-1,2,3,6-tetrahydropyrimidin,
das 1-Cyclopropyl-3-(6-chlorpyridin-3-ylmethyl)-4-dimethylamino-5-nitro-1,2,3,6-tetrahydropyrimidin,
das 1-Methyl-3-(6-chlorpyridin-3-ylmethyl)-4-dimethylamino-5-nitro-1,2,3,6-tetrahydropyrimidin,
das 1-Aethyl-3-methyl-4-[(methyl-)(pyridin-3-ylmethyl)]-amino-5-nitro-1,2,3,6-tetrahydropyrimidin,
das 1-Aethyl-3-isobutyl-4-[(cyclopropyl-)(pyridin-3-ylmethyl)]-amino-5-nitro-1,2,3,6-tetrahydropyrimidin,
das 1-Cyclopropyl-3-methyl-4-[(methyl-)(6-chlorpyridin-3-ylmethyl)]-amino-5-nitro-1,2,3,6-tetrahydropyrimidin,
das 1-Cyclopropyl-3-methyl-4-[(äthyl-)(6-chlorpyridin-3-ylmethyl)]-amino-5-nitro-1,2,3,6-tetrahydropyrimidin,
das 1-Methoxycarbonylmethyl-3-methyl-4-[(äthyl-)(6-chlorpyridin-3-yl-methyl)]-amino-5-nitro-1,2,3,6-tetrahydropyrimidin,
das 1-Methoxycarbonylmethyl-3-methyl-4-[(methyl-)(2,6-dichlorpyridin-4-ylmethyl)]-amino-5-nitro-1,2,3,6-tetrahydropyrimidin,
das 1-Cyclopropyl-3-(2,6-dichlorpyridin-4-ylmethyl)-4-dimethylamino-5-nitro-1,2,3,6-tetrahydropyrimidin,
das 1-Cyclopropyl-3-methyl-4-[(methyl-)(6-trifluormethylpyridin-3-yl-methyl)]-amino-5-nitro-1,2,3,6-tetrahydropyrimidin und
das 1-Methoxycarbonylmethyl-3-(6-trifluormethylpyridin-3-ylmethyl)-4-dimethylamino-5-nitro-1,2,3,6-tetrahydropyrimidin.

Die erfindungsgemässen Verbindungen der Formel I können hergestellt werden, indem man ein Nitroenamin der Formel II mit einem primären Amin der Formel III und mit zwei Mol Formaldehyd

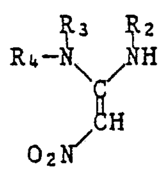

(II)

+ $2CH_2O + H_2NR_1$ (III)

I + $2H_2O$

umsetzt, wobei die Reste $R_1$, $R_2$, $R_3$ und $R_4$ die für die Verbindung der Formel I angegebene Bedeutungen haben und das Reaktionsprodukt isoliert. Dieses Verfahren ist ebenfalls Gegenstand der vorliegenden Erfindung.

Die verbindungen der Formel II sind zum Teil bekannt und können nach bekannten Methoden, wie beispielsweise durch stufenweise Umsetzung von 1-Nitro-2,2-dimethylthioäthylen mit Aminen der Formeln $R_2NH_2$ und $R_3NHR_4$, hergestellt werden (EP-A 302,833).

Die Umsetzung kann ohne Lösungsmittel, oder in Wasser oder in einem organischen Lösungsmittel durchgeführt werden.

Die Umsetzungen verlaufen im Temperaturbereich zwischen 0° und dem Kochpunkt des Reaktionsgemisches, unter atmosphärischem oder gegebenenfalls unter erhöhtem Druck.

Die verbindungen der Formel

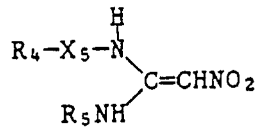

in welcher
$R_4$ für einen Aryl- oder Heteroarylrest steht, der gegebenenfalls substituiert ist,
$R_5$ für geradkettiges, verzweigtes oder cyclisches Alkyl oder Alkenyl, das gegebenenfalls durch Alkoxy oder Cycloalkyl substituiert ist, steht oder für einen gegebenenalls substituierten Aryl- oder Heteroarylrest steht,
$X_5$ für gegebenenfalls alkylsubstituiertes Methylen oder eine chemische Bindung steht, wobei $R_5$ nicht gleich Aryl ist, wenn $X_5$ eine Einfachbindung darstellt, sind als kreislaufbeeinflussende Arzneimittel, insbesondere als blutdrucksenkende Mittel aus der DE-OS 32 32 462 bekannt.

In der EP-A 154 178 werden insektizid, mitizid und nematizid wirksame 1-Pyridinylalkyl-2-nitromethyliden-1,3-diazacycloalkane beschrieben, wobei der Heterocyclus 5-7 Glieder aufweist. Diese Veröffentlichung betrifft Verbindungen, welche der Formel

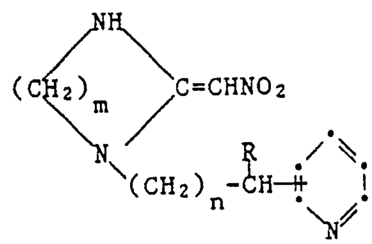

entsprechen, wobei R für ein Wasserstoffatom oder eine niedere Alkyl-Gruppe, m für 2, 3 oder 4 und n für 0, 1, 2 oder 3 steht.

Aehnliche, ebenfalls insektizid wirksame 1-Pyridinylmethyl-2-nitromethyliden-azacycloalkane und 1-Pyridinylmethyl-2-nitromethyliden-1,3-diazacycloalkane wurden auch in der EP-A 192 060 beschrieben.

Als Unterschied zu den in diesen Publikationen beschriebenen verbindungen, bei welchen das 2-ständige Aethylen-Kohlenstoffatom als Glied eines Heterocyclus definiert ist, ist der entsprechende Rest der erfindungsgemässen Verbindungen nicht Glied eines Perhydroimidazol- oder Perhydropyrimidinringes.

Andere, ebenfalls insektizid wirksame Nitroäthylenderivate sind aus den "Advances in Pesticide Science", Part 2, Pergamon Press, 1979, S. 206-217 bekannt. Diese Verbindungen enthalten jedoch keinen Pyridinylmethylrest.

In der EP-A 247 477 sind bicyclische Nitroenamine der Formel I′

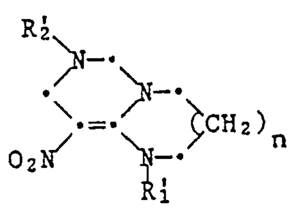

(I′)

mit Wirksamkeit als Schädlingsbekämpfungsmittel beschrieben.

Die verbindungen der Formel I gemäss vorliegender Erfindung weisen ähnlich wie die Verbindungen der Formel I′ einen Nitro-substituierten Tetrahydropyrimidinring auf, unterscheiden sich jedoch von ihnen darin, dass sie keine kondensierten Ringsysteme enthalten.

Ziel der vorliegenden Erfindung war, weitere Aktivsubstanzen für die Schädlingsbekämpfung bereitzustellen.

Ueberraschenderweise wurde gefunden, dass die erfindungsgemässen Verbindungen der Formel I wegen günstiger Warmblüter- und Pflanzenverträglichkeit wertvolle Wirkstoffe in der Schädlingsbekämpfung sind. So eignen sich die Verbindungen der Formel I z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formel I von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus- und Nutztieren, kommen vor allem Ektoparasiten, wie z.B. Milben und Zecken und Dipteren, wie z.B. Lucilia sericata in Betracht.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die verbindungen der Formel I werden in unveränderter Form oder vorzugsweise zusammen mit in der Formulierungstechnik üblichen, inerten und pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zübereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine vielzahl von granulierten Materialien anorganischer oder organischer Natur, wie insbesondere Dolomit oder zerkleinerte Pflanzenrückstände, verwendet werden.

Als oberflächenaktive verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des

Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensations produktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykolähergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die pestiziden Zübereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zübereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## 1. Herstellung der Verbindungen der Formel I

Beispiel H1. Herstellung des 1-Aethyl-3-(pyridin-3-ylmethyl)-4-dimethylamino-5-nitro-1,2,3,6-tetrahydropyrimidins (Tabelle 1, Verb. Nr. 1)

3,0 g 1-Nitro-2-dimethylamino-2-(N-pyridin-3-yl-methyl-amino)-äthylen werden mit 1,6 ml Aethylamin und 2,2 ml Formaldehyd (wässrig 37%ig), gelöst in 30 ml Aethanol, versetzt und 24 Stunden bei Raumtemperatur gerührt. Nach Eindampfen der Reaktionslösung am Rotationsverdampfer unter Wasserstrahlvakuum wird der Rückstand säulenchromatographisch über Kieselgel gereinigt. Die Säule wird mit Dichlormethan/10 % Methanol eluiert. Nach Verdampfen des Lösungsmittels in Vakuum wird das in Form von gelblichen Kristallen anfallende Endprodukt isoliert. Smp.: 97-98° C

Beispiel H2. Herstellung des 1-Aethyl-3-methyl-4-[(methyl-)(pyridin-3-ylmethyl)]-amino-5-nitro-1,2,3,6-tetrahydropyrimidins (Tabelle 1, Verb. Nr. 12)

3,0 g 1-Nitro-2-methylamino-2-[(methyl-)(pyridin-3-ylmethyl)]-aminoäthylen werden mit 1,0 g Athylamin (70%ig in $H_2O$) und 2,1 g Formaldehyd (37%ig wässrig), gelöst in 30 ml Aethanol, versetzt und während 24 Std. gerührt. Nach Eindampfen des Reaktionsgemisches am Rotationsverdampfer unter vakuum wird der

Rückstand säulenchromatographisch über Kieselgel gereinigt; die Säule wird mit Dichlormethan/10 % Methanol eluiert. Nach Verdampfen des Lösungsmittels in Vakuum wird das Titelprodukt isoliert. Smp.: 116-118 °C.

H3. Herstellung des 1-Cyclopropyl-3-methyl-4-[(methyl-)(6-chlorpyridin-3-ylmethyl)]-amino-5-nitro-1,2,3,6-tetrahydropyrimidin (Tabelle 1, Verb. Nr. 28)

Zu 2,0 g 1-Nitro-2-methylamino-2-[(methyl-)(6-chlorpyridin-3-ylmethyl)]aminoäthylen gelöst ind 20 ml Aethanol, werden bei 20 °C 1,25 ml Cyclopropylamin und anschliessend 2,35 ml 37%iges wässriges Formaldehyd zugegeben. Nach 50 Stunden Rühren bei Raumtemperatur wird das Lösungsmittel am Vakkum verdampft und das resultierende 3,5 g Rohprodukt vom Smp. 131-133 °C wird durch Chromatographie an Kieselgel mittels Dichloromethan/3 % Methanol gereinigt. Das reine Titelprodukt schmilzt bei 141-143 °C.

H4. Herstellung von 1-Cyclopropyl-3-methyl-4-[(äthyl-)(6-chlorpyridin-3-ylmethyl)]-amino-5-nitro-1,2,3,6-tetrahydropyrimidin (Tabelle 1, Verb. Nr. 29)

Zu 2,5 g 1-Nitro-2-methylamino-2-[(äthyl-)(6-chlorpyridin-3-ylmethyl)]amino-äthylen gelöst in 25 ml Aethanol werden 1,72 ml Cyclopropylamin und anschliessend 3,21 ml 37%iges wässriges Formaldehyd zugegeben. Nach 40 Stunden Rühren bei Raumtemperatur wird das Lösungsmittel in Vakuum verdampft und das resultierende Oel an Kieselgel mittels Dichloromethan/3-4 % Methanol chromatographiert. Nach Verdampfen des Lösungsmittels werden 2,6 g kristallines Titelprodukt erhalten. Smp. 122-124 °C.

H5. Herstellung von 1-Carbomethoxymethylen-3-methyl-4-[(äthyl-)(6-chlorpyridin-3-ylmethyl)]-amino-5-nitro-1,2,3,6-tetrahydropyrimidin (Tabelle 1, Verb. Nr. 30)

Zu 3,4 g 1-Nitro-2-methylamino-2-[(äthyl-)(6-chlorpyridin-3-ylmethyl)]amino-äthylen, gelöst in 34 ml Aethanol, werden 3,64 g Glycinmethylesterhydrochlorid, dann 2 ml Triäthylamin und schliesslich 3,8 ml 37%iges Formaldehyd zugesetzt. Nach 40 Stunden Rühren wird das Lösungsmittel in Vakuum verdampft und der Rückstand wird mit Dichloromethan/3 % Methanol an Kieselgel chromatographisch gereingt. Nach Verdampfen des Lösungsmittels in Vakuum resultieren 1,8 g zähflüssiges Oel, dessen Einheitlichkeit dünnschichtchromatographisch und mit Hilfe von NMR-Spektren nachzuweisen ist.
NMR-Daten (60 MHz, $CDCl_3$, TMS). 1,2 ppm: 3H, Triplett; 3,05 ppm: 3H, Singulett, O-Methyl; 3,4- ppm: 3H, Singulett, N-Methyl; 3,7 ppm: 6H, Multiplett; 4,3 ppm: 2H, Dublett,; 7,1-8,4 ppm: 3H, Multiplett.

H6. Herstellung von 1-Aethyl-3-methyl-4-[(methyl-)(6-chlorpyridin-3-ylmethyl)]-amino-5-nitro-1,2,3,6-tetrahydropyrimidin (Tabelle 1, Verb. Nr. 32)

2,6 g 1-Nitro-2-methylamino-2-[(methyl-)(6-chlorpyridin-3-ylmethyl)]aminoäthylen werden in Analogie zum Beispiel H3 mit 1,6 ml Aethylamin und 3,75 ml Formaldehyd umgesetzt und aufgearbeitet. 1,7 g des Zielprodukts vom Smp.125-126 °C werden isoliert.

H7. Herstellung von 1-Aethyl-3-methyl-4-[(methyl-)(5,6,dichlorpyridin-3-ylmethyl)]-amino-5-nitro-1,2,3,6-tetrahydropyrimidin (Tabelle 1, Verb. Nr. 34)

2,5 g 1-Nitro-2-methylamino-2-[(methyl-)(5,6-dichlorpyridin-3-ylmethyl)]-amino-äthylen in 30 ml Aethanol werden bei 5-10 °C zunächst mit 0,8 g Aethylamin, dann nach 15 Minuten Rühren mit 2,8 ml einer 37%igen wässrigen Formaldehydlösung versetzt. Aus dem Reaktionsgemisch wird nach 18 Stunden Rühren bei 20-25 °C das Lösungsmittel in Vakuum verdampft. Der Rückstand wird durch Zugabe von Aethanol kristallisiert. Nach Umkristallisation aus Acetonitril wird 1,6 g des Titelprodukts vom Smp. 115-120 °C isoliert.

H8. Herstellung des 1-Carbomethoxymethylen-3-methyl-4-[(methyl-)(5,6-dichlorpyridin-3-ylmethyl)]-amino-5-nitro-1,2,3,6-tetrahydropyrimidins (Tabelle 1, Verb. Nr. 61)

1,5 g 1-Nitro-2-methylamino-2-[(methyl-)(5,6-dichlorpyridin-3-ylmethyl)]-amino-äthylen, suspendiert in 20 ml Aethanol, werden mit einer wässrigen Lösung von Glycinmethylester (freigesetzt aus 1,5 g Glycinmethylesterhydrochlorid mittels 0,9 ml Triäthylamin in 20 ml Wasser und durch Erwärmen während 15 Minuten auf 30 °C) versetzt. Darauf werden 1,5 ml 37%iges wässriges Formaldehyd zum Reaktionsgemisch zugegeben. Nach 16 Stunden Rühren der klaren Lösung bei Raumtemperatur werden die Lösungsmittel in Vakuum verdampft und der Rückstand wird an Kieselgel mittels Dichloromethan/10 % Methanol chromatographiert. Es resultiert 0,7 g Titelprodukt vom Smp. 68-70 °C.

H9. Herstellung des 1-Carbomethoxymethylen-3-cyclopropyl-4-[(methyl-)(5,6-dichlorpyridin-3-ylmethyl)]-amino-5-nitro-1,2,3,6-tetrahydropyrimidin (Tabelle 1, Verb. Nr. 62)

1,5 g Nitro-2-cyclopropylamino-2-[(methyl-)(5,6-dichlorpyridin-3-ylmethyl)]-amino-äthylen werden mit werden mit einer wässrigen Lösung von Glycinmethylester in Analogie zum Beispiel H8 umgesetzt und das Reaktionsgut wird ebenfalls in Analogie dazu aufgearbeitet, wobei 1,3 g des bei 132-133 °C schmelzenden Titelprodukts isoliert werden.

H10. Herstellung von 1-Propenyl-2-methyl-3-[(methyl-)(6-chlorpyridin-3-ylmethyl)]-amino-5-nitro-1,2,3,6-tetrahydropyrimidin (Tabelle 1, Verb. Nr. 64)

3,0 g 1-Nitro-2-äthylamino-2-[(methyl-)(6-chlorpyridin-3-ylmethyl)]-amino-äthylen, suspendiert in 20 ml Aethanol werden nacheinander mit 1,9 ml Allylamin und 4,5 ml 37%iges wässriges Formaldehyd versetzt. Nach 96 Stunden Rühren bei Raumtemperatur wird das Lösungsmittel aus dem Reaktionsgemisch in Vakuum verdampft und der Rückstand wird an Kieselgel mittels Dichloromethan/10 % Methanol chromatographiert. 3,0 g des Titelprodukts werden isoliert. Smp. 148-150 °C.

H11. Herstellung von 1-Methylmercaptoäthylen-3-äthyl-4-[(methyl-)(6-chlorpyridin-3-ylmethyl)]-amino-5-nitro-1,2,3,6-tetrahydropyrimidin (Tabelle 1, Verb. Nr. 66)

1,4 g 1-Nitro-2-Aethylamino-2-[(methyl-)(6-chlorpyridin-3-ylmethyl)]-amino-äthylen, suspendiert in 20 ml Aethanol, wird nacheinander mit 1 ml 2-Methylthioäthylamin (80%ig) und 1,8 g 37%igem wässrigem Formaldehyd versetzt. Nach 16 Stunden bei Raumtemperatur werden die Lösungsmittel am Vakuum verdampft und der Rückstand wird an Kieselgel mittels Dichloromethan/5-10 % Methanol chromatographiert. 1,4 g des Titelprodukts werden isoliert. Smp. 82-84 °C.

In analoger Weise können die folgenden verbindungen hergestellt werden:

Tabelle 1

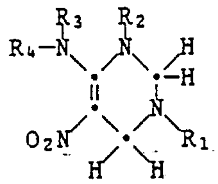

(I)

| Verb. Nr. | $R_1$ | $R_2$ | $R_3$ | $R_4$ | Physik. Konst. |
|---|---|---|---|---|---|
| 1 | $C_2H_5$ | *) | $CH_3$ | $CH_3$ | Smp.: 97-98°C |
| 2 | $i\text{-}C_4H_9$ | *) | $CH_3$ | $CH_3$ | $n_D^{24°}$: 1,5793 |
| 3 | $C_2H_5$ | *) | $n\text{-}C_4H_9$ | $CH_3$ | $n_D^{24°}$: 1,5653 |
| 4 | $i\text{-}C_3H_7$ | *) | $n\text{-}C_4H_9$ | $CH_3$ | $n_D^{24°}$: 1,5455 |
| 5 | $C_2H_5$ | *) | $CH_3$ | $CH_3$ | |
| 6 | $i\text{-}C_3H_7$ | *) | $CH_3$ | $CH_3$ | |
| 7 | $CH_2CH_2CN$ | *) | $CH_3$ | $CH_3$ | |
| 8 | $n\text{-}C_4H_9$ | *) | $CH_3$ | $CH_3$ | |
| 9 | Cyclopropyl | *) | $CH_3$ | $CH_3$ | |
| 10 | $CH_2CH_2COOCH_2CH_3$ | *) | $CH_3$ | $CH_3$ | |
| 11 | $CH_2CH_2COOH$ | *) | $CH_3$ | $CH_3$ | |
| 12 | $C_2H_5$ | $CH_3$ | *) | $CH_3$ | Smp.: 116-118°C |
| 13 | $C_2H_5$ | $i\text{-}C_4H_9$ | *) | Cyclopropyl | $n_D^{24°}$: 1,5810 |
| 14 | $i\text{-}C_3H_7$ | $CH_3$ | *) | $CH_3$ | |
| 15 | $CH_2CH_2CN$ | $CH_3$ | *) | $CH_3$ | |
| 16 | $C_6H_5\text{-}CH_2$ | $CH_3$ | *) | $C_2H_5$ | dickes Oel |
| 17 | $(CH_3)_2CHCH_2$ | $CH_3$ | *) | $C_2H_5$ | dickes Oel |
| 18 | $(CH_3)_2CHCH_2$ | $CH_3$ | *) | $CH_3$ | Smp.: 127-129°C |
| 19 | 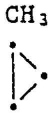 | $CH_3$ | *) | $CH_3$ | Smp.: 147-148°C |
| 20 | $CH_3$ | $CH_3$ | *) | $C_2H_5$ | Smp.: 143-149°C |
| 21 | $C_2H_5COOC_2H_5$ | $CH_3$ | *) | $CH_3$ | |
| 22 | $C_2H_5COOH$ | $CH_3$ | *) | $CH_3$ | |

*) 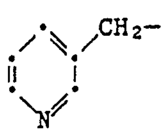

Tabelle 1 (Fortsetzung)

| Verb. Nr. | $R_1$ | $R_2$ | $R_3$ | $R_4$ | Physik. Konst. |
|---|---|---|---|---|---|
| 23 | $C_2H_5COOCH_3$ | $CH_3$ | *) | $CH_3$ | |
| 24 | $C_2H_5COOCH_3$ | *) | $CH_3$ | $CH_3$ | |
| 25 | $CH_2COOCH_3$ | $CH_3$ | *) | $CH_3$ | Smp.: 126-132°C |
| 26 | 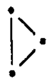 | **) | $CH_3$ | $CH_3$ | dickel Oel |
| 27 | $CH_3$ | **) | $CH_3$ | $CH_3$ | Oel |
| 28 | 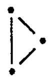 | $CH_3$ | **) | $CH_3$ | Smp.: 141-143°C |
| 29 | 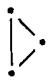 | $CH_3$ | **) | $C_2H_5$ | Smp.: 122-124°C |
| 30 | $CH_2COOCH_3$ | $CH_3$ | **) | $C_2H_5$ | fester Schaum |
| 31 | $C_2H_5$ | $C_2H_5$ | **) | $CH_3$ | Smp.: 141-144°C |
| 32 | $C_2H_5$ | $CH_3$ | **) | $CH_3$ | Smp.: 125-126°C |
| 33 | $C_2H_5$ | $CH_3$ | **) | $C_2H_5$ | Oel |
| 34 | $C_2H_5$ | $CH_3$ | ***) | $CH_3$ | Smp.: 115-120°C |
| 35 | $C_2H_5$ | $C_2H_5$ | ***) | $CH_3$ | Smp.: 150°C (Zers.) |
| 36 | $-CH_2-CH=CH_2$ | $CH_3$ | *) | $C_2H_5$ | |
| 37 | $-CH_2-CH=CH_2$ | $CH_3$ | **) | $C_2H_5$ | |
| 38 | $-CH_2-C\equiv CH_3$ | $CH_3$ | *) | $C_2H_5$ | Smp.: 121-123°C |
| 39 | $-CH_2-C\equiv CH_3$ | $CH_3$ | **) | $C_2H_5$ | |
| 40 | $-CH_2-CF_3$ | $CH_3$ | *) | $C_2H_5$ | |
| 41 | $-CH_2-CF_3$ | $CH_3$ | **) | $C_2H_5$ | |
| 42 | $-CH_2-CN$ | $CH_3$ | *) | $C_2H_5$ | |
| 43 | $-CH_2-CN$ | $CH_3$ | **) | $C_2H_5$ | |
| 44 | $-CH_2-CONH_2$ | $CH_3$ | *) | $C_2H_5$ | |
| 45 | $-CH_2-CONH_2$ | $CH_3$ | **) | $C_2H_5$ | |
| 46 | 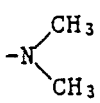 | $CH_3$ | *) | $C_2H_5$ | |
| 47 | 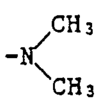 | $CH_3$ | **) | $C_2H_5$ | |
| 48 | -NHCOOEt | $CH_3$ | *) | $C_2H_5$ | |
| 49 | -NHCOOEt | $CH_3$ | **) | $C_2H_5$ | |

| Verb. Nr. | $R_1$ | $R_2$ | $R_3$ | $R_4$ | Physik. Konst. |
|---|---|---|---|---|---|
| 50 | – 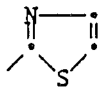 | $CH_3$ | *) | $C_2H_5$ | |
| 51 | – 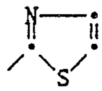 | $CH_3$ | **) | $C_2H_5$ | |
| 52 | – 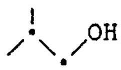 | $CH_3$ | *) | $C_2H_5$ | |
| 53 | – 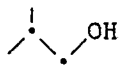 | $CH_3$ | **) | $C_2H_5$ | |
| 54 | $-CH_2CH_2OCH_3$ | $CH_3$ | *) | $C_2H_5$ | Oel |
| 55 | $-CH_2CH_2OCH_3$ | $CH_3$ | **) | $C_2H_5$ | |
| 56 | $-CH_2CH_2SCH_3$ | $CH_3$ | *) | $C_2H_5$ | |
| 57 | $-CH_2CH_2SCH_3$ | $CH_3$ | **) | $C_2H_5$ | |
| 58 | $-O-CH_2CH_3$ | $CH_3$ | *) | $C_2H_5$ | |
| 59 | $-O-CH_2CH_3$ | $CH_3$ | **) | $C_2H_5$ | |
| 60 | 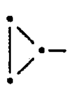 | $CH_3-$ | **) | 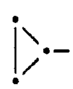 | Smp.: 142-145°C |
| 61 | $-CH_2COOCH_3$ | $CH_3-$ | ***) | $CH_3-$ | Smp.: 68-70°C |
| 62 | $-CH_2COOCH_3$ | 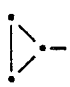 | ***) | $CH_3-$ | Smp.: 132-133°C |
| 63 | 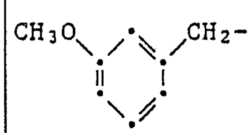 | $CH_3-$ | ***) | $CH_3-$ | Oel |
| 64 | $CH_3-CH=CH-$ | $C_2H_5-$ | **) | $CH_3-$ | Smp.: 148-150°C |
| 65 | $CH_3CH_2O-$ | $C_2H_5-$ | **) | $CH_3-$ | Oel |
| 66 | $CH_3SCH_2CH_2-$ | $C_2H_5-$ | **) | $CH_3-$ | Smp.: 82-84°C |
| 67 | $-CH_2CH_2COOC_2H_5$ | $C_2H_5-$ | **) | $CH_3-$ | Oel |
| 68 | $HOCH_2\overset{|}{C}H(CH_3)$ | $C_2H_5-$ | **) | $CH_3-$ | Oel |
| 69 | $-CH_2COOCH_3$ | $C_2H_5-$ | **) | $CH_3-$ | Oel |
| 70 | $CH_3SCH_2CH_2-$ | $CH_3-$ | **) | $CH_3-$ | Oel |

*) 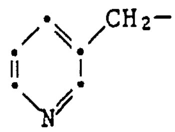

**) 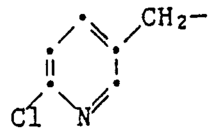

***) 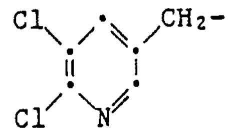

Beispiele für die Formulierungen von Wirkstoffen gemäss den Herstellungsbeispielen

(% = Gewichtsprozent)

| Beispiel F1 Emulsions-Konzentrate | a) | b) |
|---|---|---|
| Wirkstoff gemäss den Herstellungsbeispielen 1 bis 11 | 10 % | 25 % |
| Ca-Dodecylbenzolsulfonat | - | 5 % |
| Ricinusöl-polyäthylenglykoläther (36 Mol AeO) | 5 % | 5 % |
| Tributylphenol-polyäthylenglykoläther (30 Mol AeO) | 20 % | - |
| Cyclohexanon | - | 40 % |
| Butanol | 15 % | - |
| Xylolgemisch | - | 25 % |
| Essigester | 50 % | - |

Aus solchen Konzentraten können durch Verdünnen mit Wasser Emulsionen jeder gewünschten Konzentration hergestellt werden.

| Beispiel F2 Lösungen | a) | b) |
|---|---|---|
| Wirkstoff gemäss den Herstellungsbeispielen 1 bis 11 | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190° C) | - | 74 % |

Die Lösungen sind zur Anwendung in Form kleinster Tropfen geeignet.

| Beispiel F3 Granulate | a) | b) |
|---|---|---|
| Wirkstoff gemäss den Herstellungsbeispielen 1 bis 11 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| Beispiel F4 Extruder Granulat | |
|---|---|
| Wirkstoff gemäss den Herstellungsbeispielen 1 bis 11 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und it Wasser angefeuchtet. Dieses Gemisch wird extrudiert und anschliessend im Luftstrom getrocknet.

| Beispiel F5 Umhüllungs-Granulat | |
|---|---|
| Wirkstoff gemäss den Herstellungsbeispielen 1 bis 11 | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie umhüllungs-Granulate.

| Beispiel F6 Stäubemittel | a) | b) | c) | d) |
|---|---|---|---|---|
| Wirkstoff gemäss den Herstellungsbeispielen 1 bis 11 | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

| Beispiel F7 Spritzpulver | a) | b) | c) |
|---|---|---|---|
| Wirkstoff gemäss den Herstellungsbeispielen 1 bis 11 | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionenen jeder gewünschten Konzentration verdünnen lassen.

| Beispiel F8 Suspensions-Konzentrat | |
|---|---|
| Wirkstoff gemäss den Herstellungsbeispielen 1 bis 11 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1:

Frassgift- und Kontakt-Wirkung auf Laodelphax striatellus und Nilaparvata lugens (Nymphen);

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit 100 ml einer, aus dem Emulsionskonzentrat gemäss Beispiel F1) durch Wasserzusatz hergestellten Emulsion, enthaltend 400 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Die Verbindungen Nr. 1-4, 12-13 und 26-30 zeigen 80-100 % Wirkung in diesem Test gegen Nilaparvata lugens.

Beispiel B2:

Systemische Wirkung auf Nilaparvata lugens

Etwa 10 Tage alte Reispflanzen (ca. 10 cm hoch) werden in einen Plastik-Becher eingestellt, der 20 ml einer, aus dem Emulsionskonzentrat gemäss Beispiel F1) durch Wasserzusatz hergestellten, wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reispflanze wird durch ein Loch in dem Plastikdeckel in die wässrige Test-Zübereitung geschoben. Das Loch wurde dann mit Watte abgedichtet, um die Pflanze zu fixieren und den Einfluss der Gasphase aus der Test-Zübereitung auszuschalten. Dann wird die Reispflanze mit 20 Nymphen von Nilaparvata lugens im N 2 bis N 3 Stadium besiedelt und mit einem Plastikzylinder abgedeckt. Der Versuch wird bei 20° C und 60 % relativer Luftfeuchtigkeit mit einer Beleuchtungsperiode von 16 Stunden durchgeführt. Nach fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Die Verbindungen Nr. 1-4, 12-13 und 26-30 zeigen im obigem Test 80-100%ige Wirkung (Mortalität) gegen Nilaparvata lugens.

B.3 Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlüng jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23° C, bei 55 % relativer Luftfeuchtigkeit und mit einer Belichtungsperiode von 16 Stunden durchgeführt.

Die Verbindungen Nr. 1-4, 12-13 und 26-30 zeigen in diesem Test gute Wirkung.

B.4 Kontaktwirkung auf Aphis craccivora

In Töpfen angezogene 4-5 Tage alte Erbsenkeimlinge (Vicia faba) werden vor Versuchsbeginn mit je ca. 200 Individuen der Spezies Aphis craccivora besiedelt. Die so behandelten Pflanzen werden 24 Stunden später mit einer wässrigen Zübereitung enthaltend die zu prüfenden Verbindung bis zur Tropfnässe direkt besprüht. Man verwendet pro Test-Verbindung zwei Pflanzen. Eine Auswertung der erzielten Abtötungsrate erfolgt nach weiteren 24 und 72 Stunden. Der Versuch wird bei 21-22° C und einer rel. Luftfeuchtigkeit von etwa 55 % durchgeführt.

Die Verbindungen Nr. 1-4, 12-13 und 26-30 zeigen gute Wirkung in diesem Test.

B.5 Kontaktwirkung auf Myzus persicae

Etwa 4 bis 5 Tage alte, in Wasser angezogene Erbsenkeimlinge (Vicia faba) werden vor Versuchsbeginn jeweils mit ca. 200 Individuen der Spezies Myzus persicae besiedelt. Die so behandelten Pflanzen werden 24 Stunden später mit einer wässrigen Suspension enthaltend bis zu 200 ppm der zu prüfenden Verbindung bis zur Tropfnässe direkt besprüht. Man verwendet pro Testsubstanz zwei Pflanzen. Eine Auswertung der erzielten Abtötungsrate erfolgt 24 und 72 Stunden nach Applikation. Der Versuch wird bei 21-22° C und etwa 60 % relativer Luftfeuchtigkeit durchgeführt.

Die Verbindungen Nr. 1-4, 12-13 und 26-30 zeigen gute Wirkung in diesem Test.

B.6 Frassgift-Wirkung auf Spodoptera littoralis-Larven ($L_1$)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvalen Stadium besetzt. Der Versuch wird bei 26° C und ca. 50 % relativer Luftfeuchtigkeit durchgeführt. Nach 2 und 3 Tagen wird die Mortalität und nach 5 Tagen werden Entwicklungs- und Häutungsstörungen der angesetzten Larven bestimmt.

Verbindungen Nr. 1-4, 12-13 und 26-30 zeigen gute Wirkung in diesem Test.

B.7 Frassgift-Wirkung auf Spodoptera littoralis- und Heliothis virescens-Larven ($L_3$)

Eingetopfte Sojapflanzen (Topfgrösse 10 cm Durchmesser) im 4-Blatt-Stadium werden mit wässrigen Wirkstoffemulsionen besprüht, die den Wirkstoff in einer Konzentration von 400 ppm enthalten.

Nach zwei Tagen werden die behandelten Sojapflanzen mit je 10 Larven von Spodoptera littoralis und Heliothis virescens im dritten larvalen Stadium besiedelt. Der Versuch wird bei 26° C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen Nr. 1-4, 12-13 und 26-30 zeigen 80-100%ige Wirkung (Mortalität).

B.8 Wirkung gegen Anthonomus grandis (Adulte)

Zwei eingetopfte Baumwollpflanzen im 6-Blattstadium werden jeweils mit einer wässrigen benetzungsfähigen Emulsions-Zübereitung enthaltend 400 ppm des zu prüfenden Wirkstoffes besprüht. Nach dem Antrocknen des Spritzbelages (nach etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25° C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen Nr. 1-4, 12-13 und 26-30 zeigen gute Wirkung in obigem Test.

## Ansprüche

1. Nitroenamine der Formel I

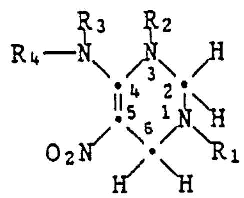

(I),

worin $R_1$ für Dialkylamino, Alkoxy, Alkenyloxy, Alkoxyalkyl, Alkylthioalkyl, Alkoxycarbonylamino, Aralkoxy, 2-Thiazolyl oder für die gegebenenfalls substituierte Reste aus der Reihe Alkyl, Alkenyl, Alkinyl, Cycloalkyl und Aralkyl steht,

$R_2$ für Nitro, Cyano, Hydroxy oder für gegebenenfalls substituierte Reste aus der Reihe Alkyl, Cycloalkyl, Alkenyl, Alkinyl, Aralkyl, Alkoxycarbonyl, Amino, Dialkylamino, Alkylcarbonyl, Alkoxycarbonyl, Alkylcarbonylamino, Alkoxycarbonylamino, Alkylcarbonyloxy und Pyridinylmethyl steht,

$R_3$ für Alkyl, Cycloalkyl oder für den gegebenenfalls substituierten Pyridinylmethylrest steht,

$R_4$ für Alkyl oder Cycloalkyl steht,

einschliesslich der Salze der verbindungen der Formel I.

2. Verbindungen gemäss Anspruch 1, worin

$R_1$ Di-$C_1$-$C_5$-alkylamino, $C_1$-$C_5$-Alkoxy, $C_3$-$C_5$-Alkenyloxy, Methoxy-$C_1$-$C_4$-alkyl, Methylthio-$C_1$-$C_4$-alkyl, Ethoxycarbonylamino, $C_7$-$C_9$-Aralkoxy, 2-Thiazolyl oder eine der mit Halogen substituierten oder unsubstituierten Reste $C_1$-$C_5$-Alkyl, $C_3$-$C_5$-Alkenyl, $C_3$-$C_5$-Alkinyl, $C_3$-$C_7$-Cycloalkyl oder $C_7$-$C_9$-Aralkyl und ferner unsubstituiertes oder an der $\alpha$- oder $\beta$-Stelle durch Cyano, Carboxy, Aminocarbonyl oder $C_1$-$C_4$-Alkoxycarbonyl substituiertes $C_1$-$C_4$-Alkyl,

$R_2$ $C_1$-$C_5$-Alkyl, $C_3$-$C_7$-Cycloalkyl, oder, falls $R_3$ für Alkyl oder Cycloalkyl steht, den Rest Z

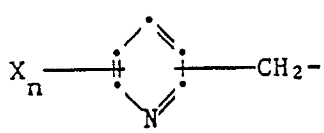

bedeutet, worin

X unabhängig voneinander für Chlor, Fluor, Nitro, Cyano, Hydroxy oder jeweils mit Halogen substituiertes oder unsubstituiertes $C_1$-$C_5$-Alkyl, $C_1$-$C_5$-Alkoxy, $C_1$-$C_5$-Alkylthio, $C_1$-$C_5$-Alkylsulfinyl oder $C_1$-$C_5$-Alkylsulfonyl und ferner $C_3$-$C_5$-Haloalkenyl, $C_1$-$C_5$-Haloalkinyl, $C_1$-$C_5$-Alkoxycarbonyl, Di-$C_1$-$C_4$-alkylamino, $C_1$-$C_5$-Alkylcarbonyl, $C_1$-$C_5$-Alkylcarbonylamino oder $C_1$-$C_5$-Alkylcarbonyloxy und n für die Zahlen 0 bis 4 steht,

$R_3$ $C_1$-$C_4$-Alkyl, $C_3$-$C_7$-Cycloalkyl oder den Rest Z, wie unter $R_2$ definiert und

$R_4$ $C_1$-$C_5$-Alkyl oder $C_3$-$C_7$-Cycloalkyl bedeuten.

3. Verbindungen gemäss Anspruch 2, worin

$R_2$ $C_1$-$C_5$-Alkyl oder $C_3$-$C_7$-Cycloalkyl,

$R_3$ Pyridin-3-ylmethyl, 6-Chlor-pyridin-3-ylmethyl, 2,6-Dichlor-pyridin-3-ylmethyl, 5,6-Dichlor-pyridin-3-ylmethyl, 6-Trifluormethyl-pyridin-3-ylmethyl oder Pyridin-4-ylmethyl und
$R_4$ $C_1$-$C_5$-Alkyl oder $C_3$-$C_7$-Cycloalkyl bedeuten,

4. Verbindungen gemäss Anspruch 1, worin $R_1$ für Dialkylamino, Alkoxy, Alkenyloxy, Aralkoxy oder für die gegebenenfalls substituierte Reste aus der Reihe Alkyl, Alkenyl, Alkinyl, Cycloalkyl, Aralkyl und Hetarylalkyl steht,
$R_2$ für Nitro, Cyano, Hydroxy oder für gegebenenfalls substituierte Reste aus der Reihe Alkyl, Cycloalkyl, Alkenyl, Alkinyl, Aralkyl, Alkoxycarbonyl, Amino, Dialkylamino, Alkylcarbonyl, Alkylcarbonylamino, Alkylcarbonyloxy und Pyridinylmethyl steht,
$R_3$ für Alkyl, Cycloalkyl oder für den gegebenenfalls substituierten Pyridinylmethylrest steht,
$R_4$ für Alkyl oder Cycloalkyl steht,
einschliesslich der Salze dieser Verbindungen.

5. Verbindungen gemäss Anspruch 4, worin
$R_1$ Dialkylamino, Alkoxy, Alkenyloxy, Aralkoxy oder einen gegebenenfalls substituierte Rest aus der Reihe Alkyl, Alkenyl, Alkinyl, Cycloalkyl, Aralkyl und Hetarylalkyl, und
$R_2$ und $R_3$ voneinander unabhängig Alkyl, Cycloalkyl oder den gegebenenfalls substituierten Pyridinylmethylrest bedeuten.

6. Verbindungen gemäss Anspruch 5, worin $R_1$ Di-$C_1$-$C_5$-alkylamino, $C_1$-$C_5$-Alkoxy, $C_3$-$C_5$-Alkenyloxy, $C_7$-$C_9$-Aralkoxy oder eine der mit Halogen substituierten oder unsubstituierten Reste $C_1$-$C_5$-Alkyl, $C_3$-$C_5$-Alkenyl, $C_3$-$C_5$-Alkinyl, $C_3$-$C_7$-Cycloalkyl oder $C_7$-$C_9$-Aralkyl und ferner unsubstituiertes oder an der $\alpha$- oder $\beta$-Stelle durch Cyano, Carboxy oder $C_1$-$C_4$-Alkoxycarbonyl substituiertes $C_1$-$C_4$-Alkyle $R_2$ $C_1$-$C_5$-Alkyl, $C_3$-$C_7$-Cycloalkyl, oder, falls $R_3$ für Alkyl oder Cycloalkyl steht, den Rest Z

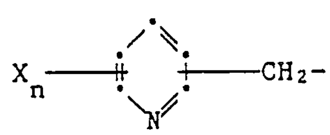

bedeutet, worin
X unabhängig voneinander für Chlor, Fluor, Nitro, Cyano, Hydroxy oder jeweils mit Halogen substituiertes oder unsubstituiertes $C_1$-$C_5$-Alkyl, $C_1$-$C_5$-Alkoxy, $C_1$-$C_5$-Alkylthio, $C_1$-$C_5$-Alkylsulfinyl oder $C_1$-$C_5$-Alkylsulfonyl und ferner $C_3$-$C_5$-Haloalkenyl, $C_3$-$C_5$-Haloalkinyl, $C_1$-$C_5$-Alkoxycarbonyl, Di-$C_1$-$C_4$-alkylamino, $C_1$-$C_5$-Alkylcarbonyl, $C_1$-$C_5$-Alkylcarbonylamino oder $C_1$-$C_5$-Alkylcarbonyloxy und n für die Zahlen 0 bis 4 steht,
$R_3$ $C_1$-$C_4$-Alkyl, $C_3$-$C_7$-Cycloalkyl oder den Rest Z, wie unter $R_2$ definiert und
$R_4$ $C_1$-$C_5$-Alkyl oder $C_3$-$C_7$-Cycloalkyl bedeuten.

7. Verbindungen gemäss Anspruch 6, worin
$R_1$ Cyclopropyl, ferner unsubstituiertes oder an der $\alpha$- oder $\beta$-Stelle durch Cyano, Carboxy oder $C_1$-$C_4$-Alkoxycarbonyl substituiertes $C_1$-$C_4$-Alkyl,
$R_2$ Pyridin-3-ylmethyl, 6-Chlor-pyridin-3-ylmethyl, 2,6-Dichlor-pyridin-3-ylmethyl, 5,6-Dichlor-pyridin-3-ylmethyl, 6-Trifluormethyl-pyridin-3-ylmethyl oder Pyridin-4-ylmethyl und
$R_3$ und $R_4$ voneinander unabhängig $C_1$-$C_5$-Alkyl oder $C_3$-$C_7$-Cycloalkyl bedeuten.

8. Verbindungen gemäss Anspruch 7, worin $R_2$ 6-Chlorpyridin-3-ylmethyl bedeutet.

9. Verbindungen gemäss Anspruch 7, worin
$R_2$ Pyridin-3-ylmethyl oder Pyridin-4-ylmethyl
$R_3$ Methyl und
$R_4$ Methyl, Aethyl, Isobutyl oder Cyclopropyl bedeuten.

10. Das 1-Aethyl-3-(pyridin-3-ylmethyl)-4-dimethylamino-5-nitro-1,2,3,6-tetrahydropyrimidin gemäss Anspruch 9.

11. Das 1-Isobutyl-3-(pyridin-3-ylmethyl)-4-dimethylamino-5-nitro-1,2,3,6-tetrahydropyrimidin gemäss Anspruch 9.

12. Das 1-Aethyl-3-(pyridin-3-ylmethyl)-4-[(methyl)(isobutyl)]-amino-5-nitro-1,2,3,6-tetrahydropyrimidin gemäss Anspruch 9.

13. Das 1-Isopropyl-3-(pyridin-3-ylmethyl)-4-[(methyl)(isobutyl)]-amino-5-nitro-1,2,3,6-tetrahydropyrimidin gemäss Anspruch 9.

14. Das 1-Cyclopropyl-3-(6-chlorpyridin-3-ylmethyl)-4-dimethylamino-5-nitro-1,2,3,6-tetrahydropyrimidin gemäss Anspruch 8.

15. Das 1-Methyl-3-(6-chlorpyridin-3-ylmethyl)-4-dimethylamino-5-nitro-1,2,3,6-tetrahydropyrimidin gemäss Anspruch 8.

16. Verbindungen der Formel I gemäss Anspruch 6, worin
$R_1$ Cyclopropyl, unsubstituiertes oder an der α- oder β-Stelle durch Cyano, Carboxy oder $C_1$-$C_4$-Alkoxycarbonyl substituiertes $C_1$-$C_4$-Alkyl,
$R_2$ $C_1$-$C_5$-Alkyl oder $C_3$-$C_7$-Cycloalkyl,
$R_3$ Pyridin-3-ylmethyl, 6-Chlor-pyridin-3-ylmethyl, 2,6-Dichlor-pyridin-3-ylmethyl, 6-Trifluormethyl-pyridin-3-ylmethyl oder Pyridin-4-ylmethyl und
$R_4$ $C_1$-$C_5$-Alkyl oder $C_3$-$C_7$-Cycloalkyl bedeuten.

17. Verbindungen gemäss Anspruch 16, worin
$R_1$ Cyclopropyl, unsubstituiertes oder an der α- oder β-Stelle durch Cyano, Carboxy, Methoxycarbonyl oder Aethoxycarbonyl substituiertes $C_1$-$C_4$-Alkyl,
$R_2$ Methyl, Aethyl, Isobutyl oder Cyclopropyl und
$R_4$ Methyl, Aethyl oder Cyclopropyl bedeuten.

18. Das 1-Aethyl-3-methyl-4-[(methyl-)(pyridin-3-ylmethyl)]-amino-5-nitro-1,2,3,6-tetrahydropyrimidin gemäss Anspruch 17.

19. Das 1-Aethyl-3-isobutyl-4-[(cyclopropyl-)(pyridin-3-ylmethyl)]-amino-5-nitro-1,2,3,6-tetrahydropyrimidin gemäss Anspruch 17.

20. Das 1-Cyclopropyl-3-methyl-4-[(methyl-)(6-chlorpyridin-3-ylmethyl)]-amino-5-nitro-1,2,3,6-tetrahydropyrimidin, gemäss Anspruch 17.

21. Das 1-Cyclopropyl-3-methyl-4-[(äthyl-)(6-chlorpyridin-3-ylmethyl)]-amino-5-nitro-1,2,3,6-tetrahydropyrimidin, gemäss Anspruch 17.

22. Das 1-Methoxycarbonylmethyl-3-methyl-4-[(äthyl-)(6-chlorpyridin-3-ylmethyl)]-amino-5-nitro-1,2,3,6-tetrahydropyrimidin, gemäss Anspruch 17.

23. Verfahren zur Herstellung einer Verbindung der Formel I, Anspruch 1, dadurch gekennzeichnet, dass man ein Nitroenamin der Formel II

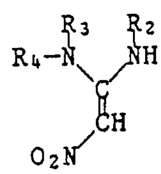

(II)

worin $R_2$, $R_3$ und $R_4$ die unter Formel I gegebenen Bedeutungen haben, mit einem primären Amin der Formel III

$H_2N$-$R_1$ (III)

worin $R_1$ die unter Formel I gegebene Bedeutung hat, und zwei Mol Formaldehyd umsetzt und das Reaktionsprodukt der Formel I isoliert.

24. Schädlingsbekämpfungsmittel, welches als aktive Komponente mindestens eine Verbindung der Formel I, gemäss Anspruch 1 enthält, zusammen mit geeigneten Trägern und/oder Zugschlagstoffen.

25. Schädlingsbekämpfungsmittel gemäss Anspruch 24, welches als aktive Komponente mindestens eine Verbindung der Formel I, gemäss den Ansprüchen 4 - 22 enthält.

26. Verwendung einer Verbindung der Formel I gemäss Anspruch 1 zur Bekämpfung von Schädlingen an Tieren und Pflanzen.

27. Verfahren zur Bekämpfung von Schädlingen an Tieren und Pflanzen, dadurch gekennzeichnet, dass man die Schädlinge in ihren verschiedenen Entwicklungsstadien mit einer Verbindung der Formel I gemäss Anspruch 1 in Kontakt bringt.

Patentansprüche für folgenden Vertragsstaat: ES

1. Verfahren zur Herstellung von Nitroenaminen der Formel I

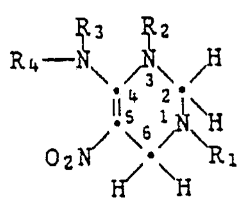

(I),

worin $R_1$ für Dialkylamino, Alkoxy, Alkenyloxy, Alkoxyalkyl, Alkylthioalkyl, Alkoxycarbonylamino, Aralkoxy, 2-Thiazolyl oder für die gegebenenfalls substituierte Reste aus der Reihe Alkyl, Alkenyl, Alkinyl, Cycloalkyl und Aralkyl steht,
$R_2$ für Nitro, Cyano, Hydroxy oder für gegebenenfalls substituierte Reste aus der Reihe Alkyl, Cycloalkyl, Alkenyl, Alkinyl, Aralkyl, Alkoxycarbonyl, Amino, Dialkylamino, Alkylcarbonyl, Alkoxycarbonyl, Alkylcarbonylamino, Alkoxycarbonylamino, Alkylcarbonyloxy und Pyridinylmethyl steht,
$R_3$ für Alkyl, Cycloalkyl oder für den gegebenenfalls substituierten Pyridinylmethylrest steht,
$R_4$ für Alkyl oder Cycloalkyl steht,
einschliesslich der Salze der Verbindungen der Formel I, dadurch gekennzeichnet, dass man ein Nitroenamin der Formel II

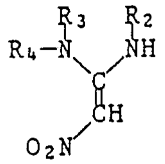

(II)

worin $R_2$, $R_3$ und $R_4$ die unter Formel I gegebenen Bedeutungen haben, mit einem primären Amin der Formel III
$H_2N\text{-}R_1$ (III)
worin $R_1$ die unter Formel I gegebene Bedeutung hat, und zwei Mol Formaldehyd umsetzt und das Reaktionsprodukt der Formel I isoliert.

2. Verfahren gemäss Anspruch 1, worin $R_1$ Di-$C_1$-$C_5$-alkylamino, $C_1$-$C_5$-Alkoxy, $C_3$-$C_5$-Alkenyloxy, Methoxy-$C_1$-$C_4$-alkyl, Methylthio-$C_1$-$C_4$-alkyl, Ethoxycarbonylamino, $C_7$-$C_9$-Aralkoxy, 2-Thiazolyl oder eine der mit Halogen substituierten oder unsubstituierten Reste $C_1$-$C_5$-Alkyl, $C_3$-$C_5$-Alkenyl, $C_3$-$C_5$-Alkinyl, $C_3$-$C_7$-Cycloalkyl oder $C_7$-$C_9$-Aralkyl und ferner unsubstituiertes oder an der $\alpha$- oder $\beta$-Stelle durch Cyano, Carboxy, Aminocarbonyl oder $C_1$-$C_4$-Alkoxycarbonyl substituiertes $C_1$-$C_4$-Alkyl,
$R_2$ $C_1$-$C_5$-Alkyl, $C_3$-$C_7$-Cycloalkyl, oder, falls $R_3$ für Alkyl oder Cycloalkyl steht, den Rest Z

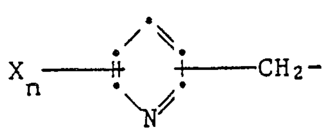

bedeutet, worin
X unabhängig voneinander für Chlor, Fluor, Nitro, Cyano, Hydroxy oder jeweils mit Halogen substituiertes oder unsubstituiertes $C_1$-$C_5$-Alkyl, $C_1$-$C_5$-Alkoxy, $C_1$-$C_5$-Alkylthio, $C_1$-$C_5$-Alkylsulfinyl oder $C_1$-$C_5$-Alkylsulfonyl und ferner $C_3$-$C_5$-Haloalkenyl, $C_1$-$C_5$-Haloalkinyl, $C_1$-$C_5$-Alkoxycarbonyl, Di-$C_1$-$C_4$-alkylamino, $C_1$-$C_5$-Alkylcarbonyl, $C_1$-$C_5$-Alkylcarbonylamino oder $C_1$-$C_5$-Alkylcarbonyloxy und n für die Zahlen 0 bis 4 steht,
$R_3$ $C_1$-$C_4$-Alkyl, $C_3$-$C_7$-Cycloalkyl oder den Rest Z, wie unter $R_2$ definiert und
$R_4$ $C_1$-$C_5$-Alkyl oder $C_3$-$C_7$-Cycloalkyl bedeuten.

3. Verfahren gemäss Anspruch 2, worin
$R_2$ $C_1$-$C_5$-Alkyl oder $C_3$-$C_7$-Cycloalkyl,
$R_3$ Pyridin-3-ylmethyl, 6-Chlor-pyridin-3-ylmethyl, 2,6-Dichlor-pyridin-3-ylmethyl, 5,6-Dichlor-pyridin-3-ylmethyl, 6-Trifluormethyl-pyridin-3-ylmethyl oder Pyridin-4-ylmethyl und
$R_4$ $C_1$-$C_5$-Alkyl oder $C_3$-$C_7$-Cycloalkyl bedeuten.

4. Verfahren gemäss Anspruch 1, worin $R_1$ für Dialkylamino, Alkoxy, Alkenyloxy, Aralkoxy oder für die gegebenenfalls substituierte Reste aus der Reihe Alkyl, Alkenyl, Alkinyl, Cycloalkyl, Aralkyl und Hetarylalkyl steht,
$R_2$ für Nitro, Cyano, Hydroxy oder für gegebenenfalls substituierte Reste aus der Reihe Alkyl, Cycloalkyl, Alkenyl, Alkinyl, Aralkyl, Alkoxycarbonyl, Amino, Dialkylamino, Alkylcarbonyl, Alkylcarbonylamino, Alkylcarbonyloxy und Pyridinylmethyl steht,
$R_3$ für Alkyl, Cycloalkyl oder für den gegebenenfalls substituierten Pyridinylmethylrest steht,
$R_4$ für Alkyl oder Cycloalkyl steht,
einschliesslich der Salze dieser verbindungen.

5. Verfahren gemäss Anspruch 4, worin

$R_1$ Dialkylamino, Alkoxy, Alkenyloxy, Aralkoxy oder einen gegebenenfalls substituierte Rest aus der Reihe Alkyl, Alkenyl, Alkinyl, Cycloalkyl, Aralkyl und Hetarylalkyl, und

$R_2$ und $R_3$ voneinander unabhängig Alkyl, Cycloalkyl oder den gegebenenfalls substituierten Pyridinylmethylrest bedeuten.

6. Verfahren gemäss Anspruch 5, worin

$R_1$ Di-$C_1$-$C_5$-alkylamino, $C_1$-$C_5$-Alkoxy, $C_3$-$C_5$-Alkenyloxy, $C_7$-$C_9$-Aralkoxy oder eine der mit Halogen substituierten oder unsubstituierten Reste $C_1$-$C_5$-Alkyl, $C_3$-$C_5$-Alkenyl, $C_3$-$C_5$-Alkinyl, $C_3$-$C_7$-Cycloalkyl oder $C_7$-$C_9$-Aralkyl und ferner unsubstituiertes oder an der $\alpha$- oder $\beta$-Stelle durch Cyano, Carboxy oder $C_1$-$C_4$-Alkoxycarbonyl substituiertes $C_1$-$C_4$-Alkyl, $R_2$ $C_1$-$C_5$-Alkyl, $C_3$-$C_7$-Cycloalkyl, oder, falls $R_3$ für Alkyl oder Cycloalkyl steht, den Rest Z

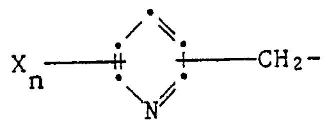

bedeutet, worin

X unabhängig voneinander für Chlor, Fluor, Nitro, Cyano, Hydroxy oder jeweils mit Halogen substituiertes oder unsubstituiertes $C_1$-$C_5$-Alkyl, $C_1$-$C_5$-Alkoxy, $C_1$-$C_5$-Alkylthio, $C_1$-$C_5$-Alkylsulfinyl oder $C_1$-$C_5$-Alkylsulfonyl und ferner $C_3$-$C_5$-Haloalkenyl, $C_3$-$C_5$-Haloalkinyl, $C_1$-$C_5$-Alkoxycarbonyl, Di-$C_1$-$C_4$-alkylamino, $C_1$-$C_5$-Alkylcarbonyl, $C_1$-$C_5$-Alkylcarbonylamino oder $C_1$-$C_5$-Alkylcarbonyloxy und n für die Zahlen 0 bis 4 steht,

$R_3$ $C_1$-$C_4$-Alkyl, $C_3$-$C_7$-Cycloalkyl oder den Rest Z, wie unter $R_2$ definiert und

$R_4$ $C_1$-$C_5$-Alkyl oder $C_1$-$C_7$-Cycloalkyl bedeuten.

7. Verfahren gemäss Anspruch 6, worin

$R_1$ Cyclopropyl, ferner unsubstituiertes oder an der $\alpha$- oder $\beta$-Stelle durch Cyano, Carboxy oder $C_1$-$C_4$-Alkoxycarbonyl substituiertes $C_1$-$C_4$-Alkyl,

$R_2$ Pyridin-3-ylmethyl, 6-Chlor-pyridin-3-ylmethyl, 2,6-Dichlor-pyridin-3-ylmethyl, 5,6-Dichlor-pyridin-3-ylmethyl, 6-Trifluormethyl-pyridin-3-ylmethyl oder Pyridin-4-ylmethyl und

$R_3$ und $R_4$ voneinander unabhängig $C_1$-$C_5$-Alkyl oder $C_3$-$C_7$-Cycloalkyl bedeuten.

8. Verfahren gemäss Anspruch 7, worin $R_2$ 6-Chlorpyridin-3-ylmethyl bedeutet.

9. Verfahren gemäss Anspruch 7, worin

$R_2$ Pyridin-3-ylmethyl oder Pyridin-4-ylmethyl

$R_3$ Methyl und

$R_4$ Methyl, Aethyl, Isobutyl oder Cyclopropyl bedeuten.

10. Verfahren zur Herstellung von 1-Aethyl-3-(pyridin-3-ylmethyl)-4-dimethylamino-5-nitro-1,2,3,6-tetrahydropyrimidin gemäss Anspruch 9.

11. Verfahren zur Herstellung von 1-Isobutyl-3-(pyridin-3-ylmethyl)-4-dimethylamino-5-nitro-1,2,3,6-tetrahydropyrimidin gemäss Anspruch 9.

12. Verfahren zur Herstellung von 1-Aethyl-3-(pyridin-3-ylmethyl)-4-[(methyl)(isobutyl)]-amino-5-nitro-1,2,3,6-tetrahydropyrimidin gemäss Anspruch 9.

13. Verfahren zur Herstellung von 1-Isopropyl-3-(pyridin-3-ylmethyl)-4-[(methyl)(isobutyl)]-amino-5-nitro-1,2,3,6-tetrahydropyrimidin gemäss Anspruch 9.

14. Verfahren zur Herstellung von 1-Cyclopropyl-3-(6-chlorpyridin-3-ylmethyl)-4-dimethylamino-5-nitro-1,2,3,6-tetrahydropyrimidin gemäss Anspruch 8.

15. Verfahren zur Herstellung von 1-Methyl-3-(6-chlorpyridin-3-ylmethyl)-4-dimethylamino-5-nitro-1,2,3,6-tetrahydropyrimidin gemäss Anspruch 8.

16. Verfahren gemäss Anspruch 6, worin

$R_1$ Cyclopropyl, unsubstituiertes oder an der $\alpha$- oder $\beta$-Stelle durch Cyano, Carboxy oder $C_1$-$C_4$-Alkoxycarbonyl substituiertes $C_1$-$C_4$-Alkyl,

$R_2$ $C_1$-$C_5$-Alkyl der $C_3$-$C_7$-Cycloalkyl,

$R_3$ Pyridin-3-ylmethyl, 6-Chlor-pyridin-3-ylmethyl, 2,6-Dichlor-pyridin-3-ylmethyl, 6-Trifluormethyl-pyridin-3-ylmethyl oder Pyridin-4-ylmethyl und

$R_4$ $C_1$-$C_5$-Alkyl oder $C_3$-$C_7$-Cycloalkyl bedeuten.

17. Verfahren gemäss Anspruch 16, worin

$R_1$ Cyclopropyl, unsubstituiertes oder an der $\alpha$- der $\beta$-Stelle durch Cyano, Carboxy, Methoxycarbonyl oder Aethoxycarbonyl substituiertes $C_1$-$C_4$-Alkyl,

$R_2$ Methyl, Aethyl, Isobutyl oder Cyclopropyl und

$R_4$ Methyl, Aethyl oder Cyclopropyl bedeuten.

18. Verfahren zur Herstellung von 1-Aethyl-3-methyl-4-[(methyl-)(pyridin-3-ylmethyl)]-amino-5-nitro-1,2,3,6-tetrahydropyrimidin gemäss Anspruch 17.

19. Verfahren zur Herstellung von 1-Aethyl-3-isobutyl-4-[(cyclopropyl-)(pyridin-3-ylmethyl)]-amino-5-nitro-1,2,3,6-tetrahydropyrimidin gemäss Anspruch 17.

20. Verfahren zur Herstellung von 1-Cyclopropyl-3-methyl-4-[(methyl-)(6-chlorpyridin-3-ylmethyl)]-amino-5-nitro-1,2,3,6-tetrahydropyrimidin, gemäss Anspruch 17.

21. Verfahren zur Herstellung von 1-Cyclopropyl-3-methyl-4-[(äthyl-)(6-chlorpyridin-3-ylmethyl)]-amino-5-nitro-1,2,3,6-tetrahydropyrimidin, gemäss Anspruch 17.

22. Verfahren zur Herstellung von 1-Methoxycarbonylmethyl-3-methyl-4-[(äthyl-)(6-chlorpyridin-3-ylmethyl)]-amino-5-nitro-1,2,3,6 tetrahydropyrimidin, gemäss Anspruch 17.